# EUROPEAN PATENT APPLICATION

(11) **EP 4 491 215 A1**
(43) Date of publication of application: **15.01.2025**
(21) Application number: 23184381.4
(22) Date of filing: 10.07.2023
(51) Int. Cl.: A61N 1/372, A61N 1/375

(54) **IMPLANT COMMUNICATION SYSTEM, MANUFACTURING METHOD AND IMPLANT PROGRAMMING DEVICE**

(71) Applicant: BIOTRONIK SE & Co. KG, 12359 Berlin (DE)
(72) Inventor: Deutschmann, Hendrik, 10247 Berlin (DE); Müller, Jens, 14195 Berlin (DE); Krüger, Daniel, 15741 Bestensee (DE)
(74) Representative: Biotronik Corporate Services SE

(57) **Abstract**

The invention relates to an implant communication system, comprising an implantable medical device (10), and a RFID-Tag (12) associated with the implantable medical device (10), wherein the RFID-Tag (12) is configured to store device information (D) of the implantable medical device (10). Furthermore, the invention relates to method for manufacturing an implant communication system, a method of operating an implant communication system and an implant programming device (20).

## Description

The invention relates to an implant communication system, a method for manufacturing an implant communication system, a method of operating an implant communication system and an implant programming device.

WO 2007/070669 A2 discloses a patient management device for portably interfacing with a plurality of implantable medical devices and method thereof is presented. Permission to interrogate one or more implantable medical devices is authenticated. Patient device data is individually exchanged through interrogation of at least one authenticated implantable medical device through short range telemetry. External device data is exchanged via communication with at least one external device through long range telemetry. At least one of the patient device and external device data is maintained contemporaneously to execution of operations to perform one or more of relay, processing, and outputting of the patient device and external device data subsequent to the interrogation of the implantable medical device.

Conventional cardiac rhythm management implants do not communicate with consumer devices such as a mobile communication device or a tablet in order to share information. The cardiac rhythm management implant communicates via coil telemetry or RF-communication which is not available as standard consumer hardware. The information stored on the implant is thus only available to medical staff having access to a programmer or to a relay device.

If a patient wearing an implantable medical device is admitted to a hospital and/or medical practice due to an acute medical condition data is read out from the implantable medical device by means of said programmer by a trained expert and/or medical practitioner in order to evaluate medical parameters of the patient recorded by the implantable medical device as well as technical parameters of the implantable medical device in order to evaluate if the implantable medical device is operating normally or whether a technical issue is present.

Programmers however are not universally available at all medical facilities such as hospitals and/or medical practices. Furthermore, such programmers may be difficult to use for untrained medical staff, acceptance of such devices is generally limited. Lately, the newest generation of cardiac rhythm management implants allow communication via Bluetooth. Usually, this is done manufacturer specific or even implant type/implant family specific.

Moreover, the information stored on Bluetooth enabled implants is generally not easily accessible since it requires access to a relay device or a patient communication device capable of communicating with the implantable medical device.

For the patient, it is however important to have necessary information about the implant available as an accessory such as an implant ID indicated on a plastic card/sticker that the patient needs to carry in a wallet or as an app on the mobile communication device. In case of a medical emergency, for patients with strokes, Alzheimer or elderly patients in general, this information might not be available for medical staff.

Said accessories such as a plastic card can be easily forgotten by the patient or overseen by medical staff or in case of an emergency, the patient may not be able to communicate said information or have no access to it.

It is therefore an object of the present invention to provide an improved implant communication system that is usable not only by medical practitioners but also by untrained medical staff and is generally widely accepted and universally available capable of accessing necessary information about the implant.

The object is solved by an implant communication system having the features of claim 1.

Furthermore, the object is solved by a method for manufacturing an implant communication system having the features of claim 11. In addition, the object is solved by a method of operating an implant communication system having the features of claim 14.

Moreover, the object is solved by an implant programming device having the features of claim 15.

Further developments and advantageous embodiments are defined in the dependent claims.

The present invention provides an implant communication system, comprising an implantable medical device, and a RFID-Tag associated with the implantable medical device, wherein the RFID-Tag is configured to store device information of the implantable medical device.

Furthermore, the present invention provides a method for manufacturing an implant communication system, comprising the steps of providing an implantable medical device, and providing a RFID-Tag associated with the implantable medical device, wherein the RFID-Tag is configured to store device information of the implantable medical device.

Moreover, the present invention provides a method of operating an implant communication system, comprising the steps of providing an implantable medical device comprising a RFID-Tag, storing device information of the implantable medical device on the RFID-Tag, and reading the RFID-Tag by a RFID reader, in particular a non-medical RFID reader, when the RFID reader is placed within a communication range of the RFID-Tag.

In addition, the present invention provides an implant programming device, comprising a RFID reader configured to read a RFID-Tag associated with the implantable medical device of the implant communication system when placed within a communication range of the RFID-Tag.

An idea of the present invention is to provide medical staff with an easy to use and widely available tool for accessing a patient's implantable medical device for the purposes of reading out data related to device information of the implantable medical device.

The conventionally used proprietary device for accessing the implantable medical device is thus replaced by a consumer device RFID Reader.

The implantable medical device may be formed by a purely therapeutic implant, an implant with therapeutic and diagnostic functions and/or an implant with therapeutic and diagnostic functions.

An example of a purely therapeutic implant/implantable medical device is e.g. a stimulator/electrode for deep brain stimulation. The therapy consists of the delivery of pulse trains without collecting diagnostic data from the stimulator.

An example of a purely diagnostic implant is e.g. a cardiac rhythm monitor. The diagnostic function consists of continuous recording of the patient's ECG and automatic evaluation of abnormalities of the heart rhythm. If such are detected, an ECG recording is stored and typically automatically transmitted to a remote monitoring system.

An example of an implant with therapeutic and diagnostic functions is e.g. a cardiac pacemaker. The pacemaker is typically implanted subcutaneously in the upper right thoracic region and the electrode is placed in the patient's heart via a large vein. The therapeutic function consists of delivering stimulation pulses to trigger a cardiac action, provided there is no spontaneous cardiac action in the patient. The diagnostic function consists, for example, in the continuous recording of the patient's ECG and automatic evaluation of abnormalities of the heart rhythm. If such are detected, an ECG recording is stored and typically automatically transmitted to a remote monitoring system.

According to an aspect of the invention, the RFID-Tag is integrated into a housing of the implantable medical device. This allows for a simpler implantation of the implantable medical device since the housing of the implantable medical device is adapted to incorporate the RFID reader.

According to a further aspect of the invention, the RFID-Tag is an active RFID-Tag connected to an internal power supply of the implantable medical device. Thus, an extended communication range of the RFID-Tag can be provided.

According to a further aspect of the invention, the RFID-Tag is arranged on an outer side of a housing of the implantable medical device. This enables an easy fitting or retrofitting of the RFID-Tag to existing implantable medical devices.

According to a further aspect of the invention, the RFID-Tag is an, optionally battery assisted, passive RFID-Tag. A passive RFID-Tag is largely maintenance free and thus advantageously provides a long service life.

According to a further aspect of the invention, the implant communication system further comprises a RFID reader, in particular a non-medical RFID reader, configured to read the RFID-Tag when placed within a communication range of the RFID-Tag. Using a standard RFID reader provides the advantage that the RFID-Tag can be read by widely available consumer devices that can be used by medical and non-medical staff.

According to a further aspect of the invention, the RFID reader is integrated into an implant programming device. In this alternative embodiment the implant programming device provides the additional functionality of being able to read RFID-Tags. Thus, medical staff familiar with using implant programming devices are able to read the RFID-Tag using their customary implant programming device.

According to a further aspect of the invention, the RFID-Tag is configured to store an ID of the implantable medical device, a reference to a manufacturer of the implantable medical device, a link to a manufacturers remote service displaying information about the implantable medical device, organ donor information and/or information on a patient's health care practitioner. The information stored on the RFID-Tag thus assists medical staff to determine a possible patient treatment.

According to a further aspect of the invention, the RFID-Tag is configured to be writeable in the field for updating data stored on the RFID-Tag. In case of a change of information such as contact details of the patient's health care practitioner, said information may be updated in the field.

According to a further aspect of the invention, the implantable medical device is a cardiac rhythm management device, in particular an implantable pulse generator, an implantable cardioverter defibrillator and/or an insertable cardiac monitor. The RFID-Tag is thus usable with a wide range of implantable medical devices.

According to a further aspect of the invention, the RFID-Tag is programmed with the device information during production of the implantable medical device. This way, default information provided by the manufacturer of the implantable medical device is stored on the RFID-Tag during production, wherein additional information may be added at a later stage by a health care provider.

According to a further aspect of the invention, the RFID-Tag is integrated into a housing of the implantable medical device or the RFID-Tag is arranged on an outer side of a housing of the implantable medical device. The specific mounting and/or integration of the RFID-Tag into a housing of the implantable medical device may be determined based on a type or model of the respective implantable medical device thus providing a device specific optimal solution.

The herein described features of the implant communication system are also disclosed for the method of operating an implant communication system and vice versa.

For a more complete understanding of the present invention and advantages thereof, reference is now made to the following description taken in conjunction with the accompanying drawings. The invention is explained in more detail below using exemplary embodiments, which are specified in the schematic figures of the drawings, in which:
- Fig. 1: shows a schematic view of an implant communication system according to a preferred embodiment of the invention;
- Fig. 2: shows a method for manufacturing an implant communication system according to the preferred embodiment of the invention;
- Fig. 3: shows a method of operating an implant communication system according to the preferred embodiment of the invention; and
- Fig. 4: shows an implant programming device according to the preferred embodiment of the invention.

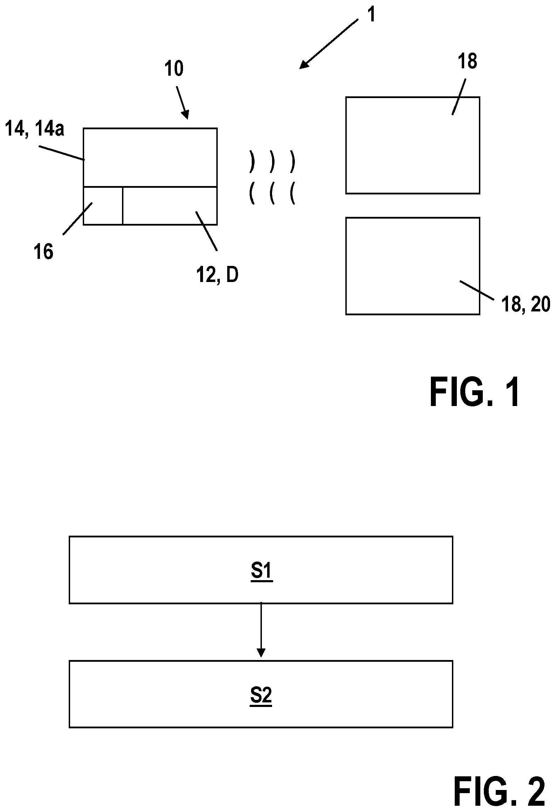

Fig. 1 shows an implant communication system 1 comprising an implantable medical device 10 and a RFID-Tag 12 associated with the implantable medical device 10, wherein the RFID-Tag 12 is configured to store device information D of the implantable medical device 10.

Furthermore, the RFID-Tag 12 is integrated into a housing 14 of the implantable medical device 10, wherein the RFID-Tag 12 is an active RFID-Tag 12 connected to an internal power supply 16 of the implantable medical device 10.

Alternatively, the RFID-Tag 12 can be arranged on an outer side 14a of a housing 14 of the implantable medical device 10. In this case, the RFID-Tag 12 is an, optionally battery assisted, passive RFID-Tag 12.

Moreover, the implant communication system 1 comprises a RFID reader 18, in particular a non-medical RFID reader 18, configured to read the RFID-Tag 12 when placed within a communication range of the RFID-Tag 12.

The RFID reader 18 is preferably a standard, standalone RFID reader. Alternatively, the RFID reader 18 can be integrated into an implant programming device 20.

The RFID-Tag 12 is configured to store an ID of the implantable medical device 10, a reference to a manufacturer of the implantable medical device 10, a link to a manufacturers remote service displaying information about the implantable medical device 10, organ donor information and/or information on a patient's health care practitioner.

In addition, the RFID-Tag 12 is configured to be writeable in the field for updating data stored on the RFID-Tag 12. The implantable medical device 10 is a cardiac rhythm management device, in particular an implantable pulse generator, an implantable cardioverter defibrillator and/or an insertable cardiac monitor.

Fig. 2 shows a method for manufacturing an implant communication system 1 according to the preferred embodiment of the invention.

The method comprises providing an implantable medical device 10. Furthermore, the method comprises providing a RFID-Tag 12 associated with the implantable medical device 10, wherein the RFID-Tag 12 is configured to store device information D of the implantable medical device 10.

The RFID-Tag 12 is programmed with the device information D during production of the implantable medical device 10.

Furthermore, the RFID-Tag 12 is integrated into a housing 14 of the implantable medical device 10 or the RFID-Tag 12 is arranged on an outer side 14a of a housing 14 of the implantable medical device 10.

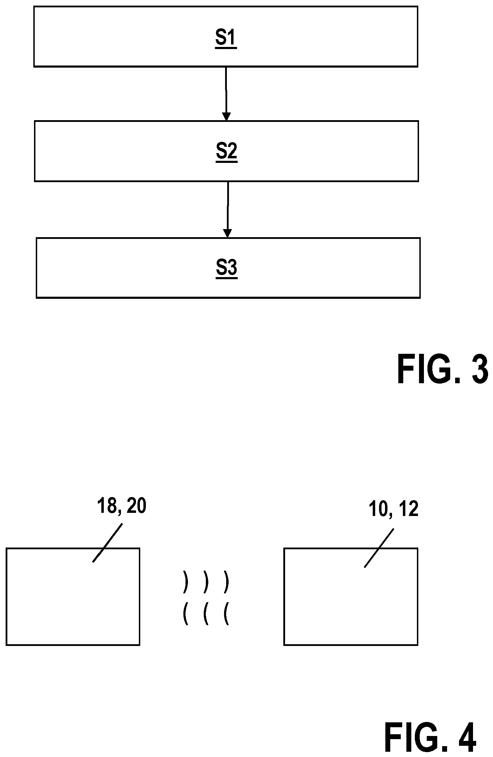

Fig. 3 shows a method of operating an implant communication system 1 according to the preferred embodiment of the invention.

The method comprises providing an implantable medical device 10 comprising a RFID-Tag 12. Furthermore, the method comprises storing device information D of the implantable medical device 10 on the RFID-Tag 12.

Moreover, the method comprises reading the RFID-Tag 12 by a RFID reader 18, in particular a non-medical RFID reader 18, when the RFID reader 18 is placed within a communication range of the RFID-Tag 12.

Fig. 4 shows an implant programming device 20 according to the preferred embodiment of the invention.

The implant programming device 20 comprises a RFID reader 18 configured to read a RFID-Tag 12 associated with the implantable medical device 10 of the implant communication system 1 when placed within a communication range of the RFID-Tag 12.

### Reference Signs

- 1: implant communication system
- 10: implantable medical device
- 12: RFID-Tag
- 14: housing
- 14a: outer side
- 16: internal power supply
- 18: RFID-Reader
- 20: implant programming device
- D: device information
- S1-S3: method steps
- S1'-S3': method steps

## Claims

1. Implant communication system (1), comprising:
an implantable medical device (10); and
a RFID-Tag (12) associated with the implantable medical device (10), wherein the RFID-Tag (12) is configured to store device information (D) of the implantable medical device (10).

2. Implant communication system of claim 1, wherein the RFID-Tag (12) is integrated into a housing (14) of the implantable medical device (10).

3. Implant communication system of claim 2, wherein the RFID-Tag (12) is an active RFID-Tag (12) connected to an internal power supply (16) of the implantable medical device (10).

4. Implant communication system of claim 1, wherein the RFID-Tag (12) is arranged on an outer side (14a) of a housing (14) of the implantable medical device (10).

5. Implant communication system of claim 4, wherein the RFID-Tag (12) is an, optionally battery assisted, passive RFID-Tag (12).

6. Implant communication system of any one of the preceding claims, wherein the implant communication system (1) further comprises a RFID reader (18), in particular a non-medical RFID reader (18), configured to read the RFID-Tag (12) when placed within a communication range of the RFID-Tag (12).

7. Implant communication system of claim 6, wherein the RFID reader (18) is integrated into an implant programming device (20).

8. Implant communication system of any one of the preceding claims, wherein the RFID-Tag (12) is configured to store an ID of the implantable medical device (10), a reference to a manufacturer of the implantable medical device (10), a link to a manufacturers remote service displaying information about the implantable medical device (10), organ donor information and/or information on a patient's health care practitioner.

9. Implant communication system of any one of the preceding claims, wherein the RFID-Tag (12) is configured to be writeable in the field for updating data stored on the RFID-Tag (12).

10. Implant communication system of any one of the preceding claims, wherein the implantable medical device (10) is a cardiac rhythm management device, in particular an implantable pulse generator, an implantable cardioverter defibrillator and/or an insertable cardiac monitor.

11. Method for manufacturing an implant communication system (1), comprising the steps of:
providing (S 1) an implantable medical device (10); and
providing (S2) a RFID-Tag (12) associated with the implantable medical device (10), wherein the RFID-Tag (12) is configured to store device information (D) of the implantable medical device (10).

12. Method of claim 11, wherein the RFID-Tag (12) is programmed with the device information (D) during production of the implantable medical device (10).

13. Method of claim 11 or 12, wherein the RFID-Tag (12) is integrated into a housing (14) of the implantable medical device (10) or the RFID-Tag (12) is arranged on an outer side (14a) of a housing (14) of the implantable medical device (10).

14. Method of operating an implant communication system (1), comprising the steps of:
providing (S1') an implantable medical device (10) comprising a RFID-Tag (12);
storing (S2') device information (D) of the implantable medical device (10) on the RFID-Tag (12); and
reading (S3') the RFID-Tag (12) by a RFID reader (18), in particular a non-medical RFID reader (18), when the RFID reader (18) is placed within a communication range of the RFID-Tag (12).

15. Implant programming device (20), comprising a RFID reader (18) configured to read a RFID-Tag (12) associated with the implantable medical device (10) of the implant communication system of any one of claims 1 to 10 when placed within a communication range of the RFID-Tag (12).
